(19)

Europäisches Patentamt
European Patent Office
Office européen des brevets

(11) **EP 3 973 866 A1**

(12) **EUROPEAN PATENT APPLICATION**

(43) Date of publication:
**30.03.2022 Bulletin 2022/13**

(21) Application number: **20198259.2**

(22) Date of filing: **25.09.2020**

(51) International Patent Classification (IPC):
**A61B 5/08** $^{(2006.01)}$       **A61B 5/00** $^{(2006.01)}$

(52) Cooperative Patent Classification (CPC):
**A61B 5/0816; A61B 5/7253; A61B 5/7257;**
A61B 2562/0219

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **SCHIPPER, Alphonsus Tarcisius Jozef Maria**
**5656 AE Eindhoven (NL)**
• **DERKX, Rene Martinus Maria**
**5656 AE Eindhoven (NL)**
• **VAN SLOUN, Ruud Johannes Gerardus**
**5656 AE Eindhoven (NL)**

(74) Representative: **Philips Intellectual Property &
Standards**
**High Tech Campus 52**
**5656 AG Eindhoven (NL)**

(54) **A PROCESSOR AND METHOD FOR DETERMINING A RESPIRATORY RATE**

(57) A processor and method for determining a breathing rate is based on processing a 3-axis acceleration signal. A gravity vector is isolated from the 3-axis acceleration signal and a coordinate transformation is performed into a transformed 3-axis coordinate system in which the isolated average gravity vector is aligned with a first axis of the transformed 3-axis coordinate system. Analysis is then performed only of the components for the remaining two axes of the transformed 3-axis coordinate system, thereby to derive a 1 dimensional respiratory signal. A respiration rate is obtained from the 1 dimensional respiratory signal using frequency analysis.

FIG. 2

EP 3 973 866 A1

**Description**

FIELD OF THE INVENTION

This invention relates to the measurement of a respiratory rate and optionally also a respiratory effort.

BACKGROUND OF THE INVENTION

The respiratory rate has proven to be a good indicator of the deterioration of the condition of a patient. In combination with other vital signs, the respiratory rate plays a crucial role in early warning systems. Therefore, there is a need for continuous and reliable monitoring of a respiration signal, from which the respiratory rate is extracted, in intensive care units of hospitals. A similar need is present in the general ward setting of hospitals and in home healthcare applications, such as in telemedicine and chronic disease management. Furthermore, the estimation of respiratory effort is important in the diagnosis of Obstructive Sleep Apnea Syndrome. The respiratory effort may be defined as the energy-consuming activity of the respiratory muscles aimed at driving respiration.

Respiration monitoring can be based on different principles: the measurement of respiratory effort, for example thorax impedance plethysmography, respiratory inductance plethysmography, accelerometers, photoplethysmography, the measurement of respiratory effect, for example sound recording, temperature sensing or carbon dioxide sensing via exhaled air via a nasal cannula, or diaphragm or parasternal muscle EMG measurement.

Some sensors are already well established to monitor respiration. In intensive care units for example, thorax impedance plethysmography is the method of choice, whereas in sleep studies respiratory inductance plethysmography, often referred to as respiration band or respiband, is also commonly used.

For ambulatory patients, such as on the general ward or in home healthcare, these sensors have limitations. A respiration band, for example, is considered to be too obtrusive and cumbersome by both medical personnel and patients. However, in many applications, ambulatory monitoring over a prolonged period is desired. A device that measures chest or rib motion is quite suitable, as it easy to wear and only requires a single point of mechanical contact with the chest. No electrical contact is required. Due to the low power consumption, the device can be quite small.

WO 2015/018752 for example discloses a method and system for obtaining a respiration signal using a chest worn device accelerometer for detecting of respiratory movements. The analysis of the movement signals involves determination of a rotation axis and/or rotation angle of that movement. A rotation model models the respiratory movement as a rotation around a single rotation axis.

It is known to use a gyroscope in addition to accelerometers, and this may be needed under certain postures, where the accelerometer does not perform well.

Respiratory rate is normally determined by either frequency analysis or time-domain analysis. Typically. some prior knowledge is assume about which directions of respiratory motion correspond to inspirations and which directions of motion correspond to expirations. However, needing this prior knowledge limits the freedom of the application. For example, both the location of attachment to the chest as well as the orientation of the sensor must be controlled in this case.

There is generally a desire to reduce the level of estimation error and therefore lack of robustness in such systems, and is it always of interest to simplify sensor designs and processing requirements. The invention has these objectives.

SUMMARY OF THE INVENTION

The invention is defined by the claims.

According to examples in accordance with an aspect of the invention, there is provided a processor for determining a breathing rate, comprising:
an input for receiving a 3-axis acceleration signal, wherein the processor is adapted to:

isolate a gravity vector from the 3-axis acceleration signal;
perform a coordinate transformation into a transformed 3-axis coordinate system in which a time-averaged gravity vector is aligned with a first axis of the transformed 3-axis coordinate system;
perform analysis of the components for the remaining two axes of the transformed 3-axis coordinate system, thereby to derive a 1 dimensional respiratory signal; and
determine a respiration rate from the 1 dimensional respiratory signal using frequency analysis.

The processing is used to analyze respiratory patterns in a 2D (horizontal) plane of the transformed 3-axis coordinate system, i.e. discarding the first (vertical) axis. This simplifies the processing. The transformed 3-axis coordinate system is a coordinate system of three orthogonal unit vectors. Similarly, the accelerometer generates a set of three orthogonal acceleration signals. By projecting the 3D-signal to the horizontal plane in this transformed coordinate space, additional robustness is gained. The number of dimensions is reduced prior to the processing to derive the breathing rate, and this reduces computational complexity.

Frequency analysis is thereby used in combination with quality-based statistical post-processing. The quality-based post-processing for example involves multiple breathing rate estimates being combined into one estimate (e.g. one per minute based on a weighted sum of 12 individual rates derived from 5 second periods). The weights may be quality numbers of the individual rates.

The processor can be used without assuming any attachment position or sensor orientation. It estimates the direction of inspirations and expirations from acceleration data captured with any orientation.

The processor may be adapted to perform pre-processing of the acceleration signal before isolating the gravity vector, wherein the pre-processing comprises sampling and low-pass filtering. This is used to extract signals of interest from the raw acceleration signal.

The processor may be adapted to isolate the gravity vector from the 3-axis acceleration signal by implementing a recursive exponential smoothing function. This provides one computationally light method for isolating the gravity vector.

The processor may perform the coordinate transformation into the transformed 3-axis coordinate system by deriving a rotation matrix which maps the isolated gravity vector to the first axis. This provides a simple computational step to align the gravity vector with one of the unit vectors of the transformed coordinate space.

The processor may be adapted to perform the analysis to derive the 1 dimensional respiratory signal using principal component analysis or using machine learning. The 1 dimensional respiratory signal is an un-calibrated estimate of the breathing effort, i.e. the instantaneous depth (and direction) of breathing.

The processor may for example be adapted to estimate the inspiratory and expiratory directions from the 1 dimensional respiratory signal. The 1 dimensional signal does not include any identification of the inspiratory and expiratory phases, but this information is needed to enable a proper analysis of the breathing characteristics of the user.

The inspiratory and expiratory directions may be estimated by determining a skewness value of the 1 dimensional respiratory signal. The skewness may for example be calculated in an iterative way.

The processor may be adapted to perform an inversion if needed, such that one type of skewness (e.g. left-skewed or right-skewed) corresponds to the inspiratory direction and another type of skewness (e.g. right-skewed or left-skewed) corresponds to the expiratory direction. This simplifies analysis of the resulting signal in that it has a known relationship with inspiratory and expiratory phases of breathing.

The processor for example determines the respiration rate from the 1 dimensional respiratory signal by applying a Fourier transform over a sliding time window and determining the frequency of the highest peak in the spectrum of each window, wherein the respiration rate is derived from the highest peaks for a set of successive windows

The invention also provides a system for determining a breathing rate, comprising:

   an accelerometer for generating a 3-axis acceleration signal; and
   the processor as defined above.

The invention also provides a method for determining a breathing rate, comprising:

   receiving a a 3-axis acceleration signal;
   isolating a gravity vector from the 3-axis acceleration signal;
   performing a coordinate transformation into a transformed 3-axis coordinate system in which a time-averaged gravity vector is aligned with a first axis of the transformed 3-axis coordinate system;
   performing analysis of the components for the remaining two axes of the transformed 3-axis coordinate system, thereby to derive a 1 dimensional respiratory signal; and
   determining a respiration rate from the 1 dimensional respiratory signal using frequency analysis.

The method is simple to implement and has good performance.

The method may comprise performing pre-processing of the acceleration signal before isolating the gravity vector, wherein the pre-processing comprises sampling and low-pass filtering.

The method may also comprise one or more of:

   isolating the gravity vector from the 3-axis acceleration signal by implementing a recursive exponential smoothing function;
   performing the coordinate transformation into the transformed 3-axis coordinate system by deriving a rotation matrix which maps the isolated gravity vector to the first axis;
   performing the analysis to derive the 1 dimensional respiratory signal using principal component analysis or using machine learning.

An iterative principal component analysis calculation is for example low in computational cost and leverages consistency over time.

The method may comprise estimating the inspiratory and expiratory directions from the 1 dimensional respiratory signal by determining a skewness value of the 1 dimensional respiratory signal.

The respiration rate may be derived from the 1 dimensional respiratory signal by applying a Fourier transform over a sliding time window and determining the frequency of the highest peak in the spectrum of each window, and deriving the respiration rate from the highest peaks for a set of successive windows

The invention may be implemented in software, and hence the invention also provides a computer program comprising computer program code which is adapted, when said computer program code is run on a computer, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows the measurement of orientation with an accelerometer;
Figure 2 shows a respiratory pattern in the accelerometer coordinate system;
Figure 3 shows the same pattern as Figure 2 in an alternative coordinate system;
Figure 4 shows a horizontal projection of the 3D acceleration pattern from Figure 2 into the horizontal plane in the alternative coordinate system;
Figure 5 shows the speed profile (the bottom pane) in relation to a reference signal (the top pane);
Figure 6 shows the processing steps which are used to process the 3D acceleration data in an example of the system and method of the invention;
Figure 7 shows an estimate of the respiratory signal;
Figure 8 shows the frequency distribution of the estimate;
Figure 9 is used to show how the respiration direction is derived; and

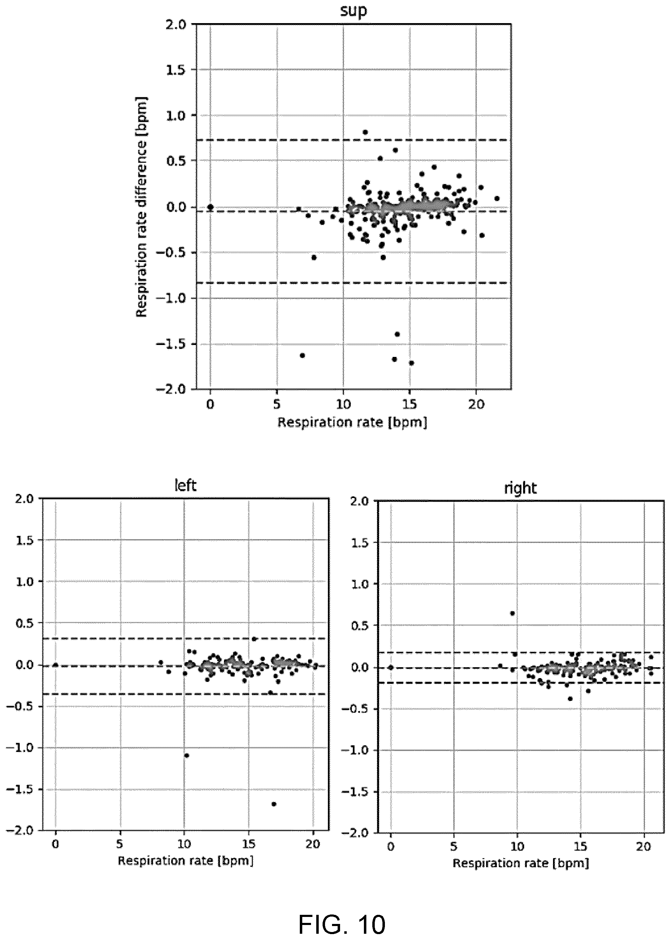

Figure 10 shows three plots to show the show the difference between the respiratory rate estimated using the processing of the invention and the respiration rate of the reference signal.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a processor, a system using the processor and a method, for determining a breathing rate by processing a 3-axis acceleration signal. A gravity vector is isolated from the 3-axis acceleration signal and a coordinate transformation is performed into a transformed 3-axis coordinate system in which the isolated gravity vector is aligned with a first axis of the transformed 3-axis coordinate system. Analysis is then performed only of the components for the remaining two axes of the transformed 3-axis coordinate system, thereby to derive a 1 dimensional respiratory signal. A respiration rate is obtained from the 1 dimensional respiratory signal using frequency analysis.

The system of the invention makes use of respiration measurements using an accelerometer. Respiration causes the sternum and ribs to rotate, and these movements are detected by the accelerometer.

During inspiration, the sternum and ribs move in a ventral and cranial direction. During expiration, the sternum and ribs move in the opposite direction. An attached accelerometer can measure this motion through orientation changes. The accelerometer operates in a quasi-static way and the projection of the gravity vector $\vec{g}$ on the axes is measured.

An accelerometer can be modeled as a box with a weight that is suspended inside of it. An accelerometer measures the force applied by the box to the weight. A 3D-accelerometer has three orthogonal axes $\{\vec{x}, \vec{y}, \vec{z}\}$, which form a right-handed coordinate system. An axis measures the projection of the acceleration on it.

If an accelerometer is in free fall, the box does not apply any force to the weight, and all axes measure zero acceleration.

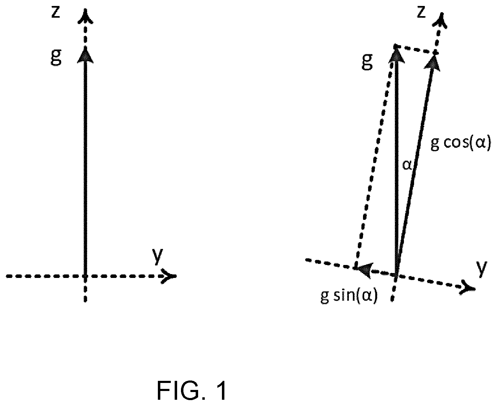

Figure 1 shows the measurement of orientation with an accelerometer. The Figure shows the axes y and z of the accelerometer. The x-axis points to the reader and is not shown. The gravity vector g is shown as g.

The left image shows the accelerometer oriented horizontally, and the z-axis is aligned with gravity. The right image shows the accelerometer tilted to the right by an angle $\alpha$. The decomposition of the gravity vector along the y- and z-axes is shown.

If an accelerometer is placed on a horizontal surface as shown in the left image, the box applies an upward force to the weight. The accelerometer measures this as an acceleration of +1 $g$ in the upward direction. If $\alpha$ is the angle between the z-axis of the accelerometer and the upward direction as shown in the right image, then $a_z = g\,cos(\alpha)$. If the upward direction is in the y-z plane then $a_y = gsin(\alpha)$.

If $\alpha$ is small then $a_z \approx g$ and $a_y \approx 0$. If $\alpha$ changes by a small amount then $\Delta a_z \approx 0$ and $\Delta a_y \approx \alpha\,g$. This is only true if the z-axis of the accelerometer points upward, which generally is not the case. As explained below, the invention makes use of a coordinate transformation to a new coordinate system $\{\vec{h_1}, \vec{h_2}, \vec{v}\}$. The new coordinate system forms an orthonormal, right-handed basis such that $\vec{v}$ points upward (aligns with $\vec{g}$). As a consequence, $\vec{h_1}$ and $\vec{h_2}$ lie in the horizontal plane (if $\vec{v}$ is defined/named as a vertical axis in the transformed coordinate system).

In such a case, it suffices to only measure the projection onto the horizontal plane. In other words, it suffices to use only the $\vec{h_1}$ and $\vec{h_2}$ components of the acceleration signal. This will only be valid if the accelerometer is operated in a quasi-static mode and if angles are small.

This approach can increase the robustness of the system. When deriving the respiratory signal, a model is used of which the parameters are estimated. By using this horizontal projection, the parameter space is constrained.

The parameters are the three coordinates of the projection axis. To obtain a one-dimensional respiratory waveform, the three-dimensional acceleration value is projected upon a projection axis. First, there is projection to the horizontal plane and then there is projection to an axis in that plane. The direction of the axis is such that increasing projection values correspond to inspiration.

Note that an accelerometer can only measure orientation changes if the projection of $\vec{g}$ on its axes changes. If the accelerometer is rotated around $\vec{g}$ then the projection on all of the accelerometer axes remains unchanged and nothing can be measured. A gyroscope is not susceptible to this as it can measure rotations irrespective of the orientation. A gyroscope is thus typically used for measuring orientation changes.

Respiratory patterns may be measured from the acceleration data, for example from an accelerometer placed on the chest of a subject.

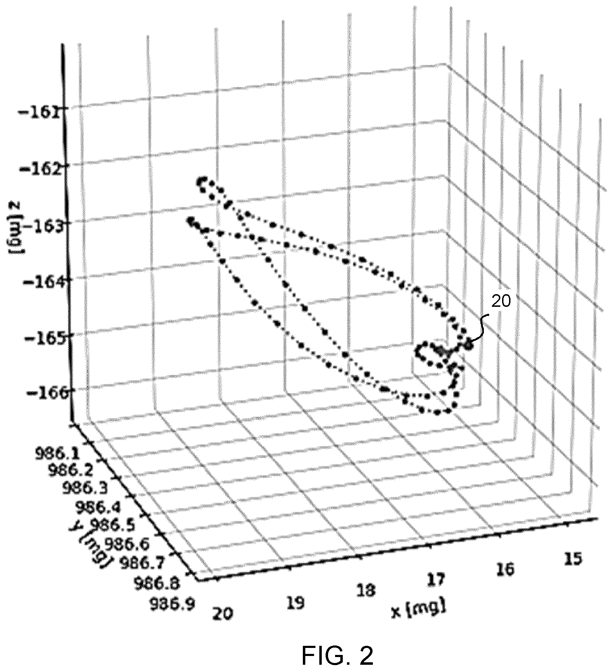

Figure 2 shows a respiratory pattern in the accelerometer coordinate system $\{\vec{x}, \vec{y}, \vec{z}\}$. The orientation of the graph is according to these axes (the z-axis is drawn vertically).

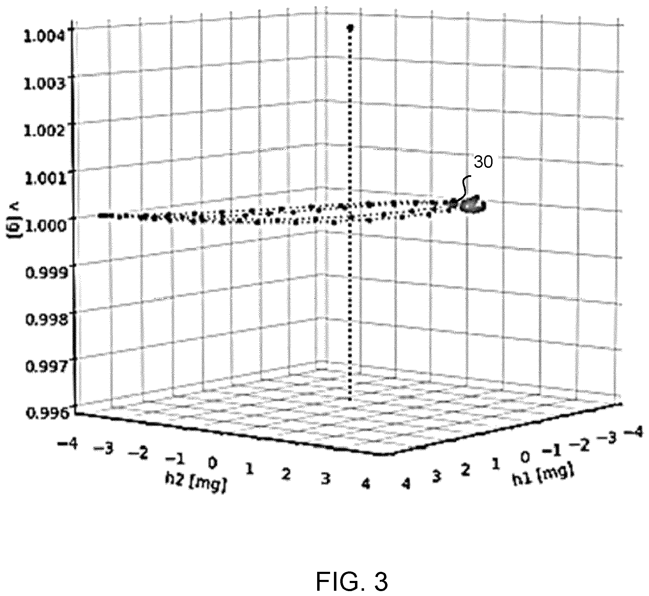

Figure 3 shows the same pattern, but in the alternative transformed coordinate system $\{\vec{h_1}, \vec{h_2}, \vec{v}\}$ explained above. In this coordinate system, $\vec{h_1}$ and $\vec{h_2}$ lie in a horizontal plane and $\vec{v}$ points upward (aligns with $\vec{g}$) and is thus the vertical line shown. It can be seen that the pattern lies in a horizontal plane, at v = 1 g.

The acceleration data for Figures 2 and 3 is sampled at 16 Hz, and the duration is 15 seconds. The data fragment spans two respiratory cycles. Each cycle forms a kind of lasso-pattern. A dotted line connects successive samples.

The dots 20, 30 indicate the start of the pattern. The axes x, y, and z are the original accelerometer axes in Figure 2, whereas Figure 3 shows the same data fragment, but then in the new coordinate system (h1, h2, v).

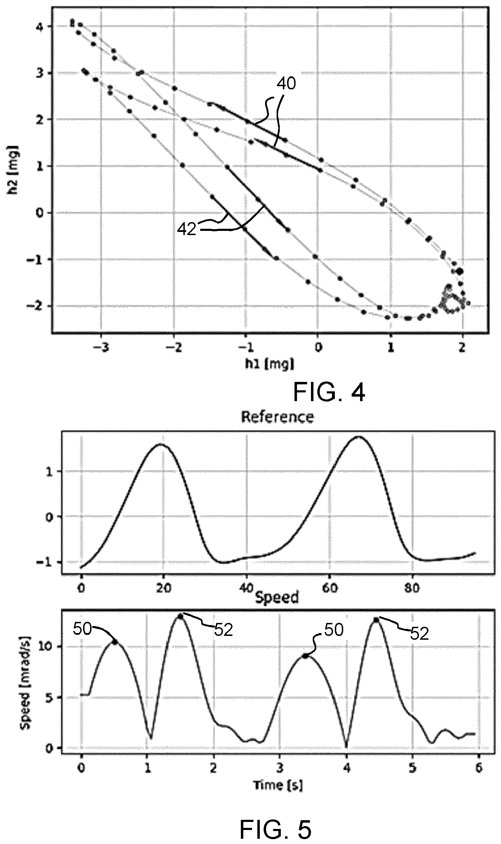

Figure 4 shows a horizontal projection of the 3D acceleration pattern from Figure 3 into the horizontal plane (h1 and h2 components), i.e. by discarding the v axis. The two respiratory cycles follow a diagonal, counter-clockwise pattern. The distance between the dots is proportional to the angular speed at which the orientation of the accelerometer changes. The further apart the dots are, the higher the speed is.

The arrows 40, 42 show the points and direction of maximum speed. The arrows 40 correspond to inspiration, and the arrows 42 correspond to expiration. Inspiration corresponds to the left and upward direction in the horizontal projection graph.

Figure 5 shows the speed profile (the bottom pane) in relation to the reference signal (the top pane).

The reference signal is an actual breathing signal as measured by other trusted sensors, and is provided for the purposes of explaining the operation of the system. It is not an available signal in the normal use of the system. The reference signal is for example obtained from a band for Respiratory Impedance Plethysmography (RIP band). Such a band is normally not used under ambulatory conditions.

The peaks in the rotational speed are at the maximum inspiratory and expiratory flow, i.e. the steepest parts of the inspiration cycle and the steepest parts of the expiration cycle in the reference signal. The peaks 50 are the inspiratory peaks and the peaks 52 are the expiratory peaks, corresponding to the vectors 40, 42.

The results presented in this application are based on a data collection study. Ten healthy subjects were used, with 80 minutes of analysis for each subject in supine, left and right postures. A chest-worn accelerometer was employed at different body positions, with voluntary breathing of the users.

The aim of the system is to obtain a respiratory signal and a respiration rate from a 3D acceleration signal.

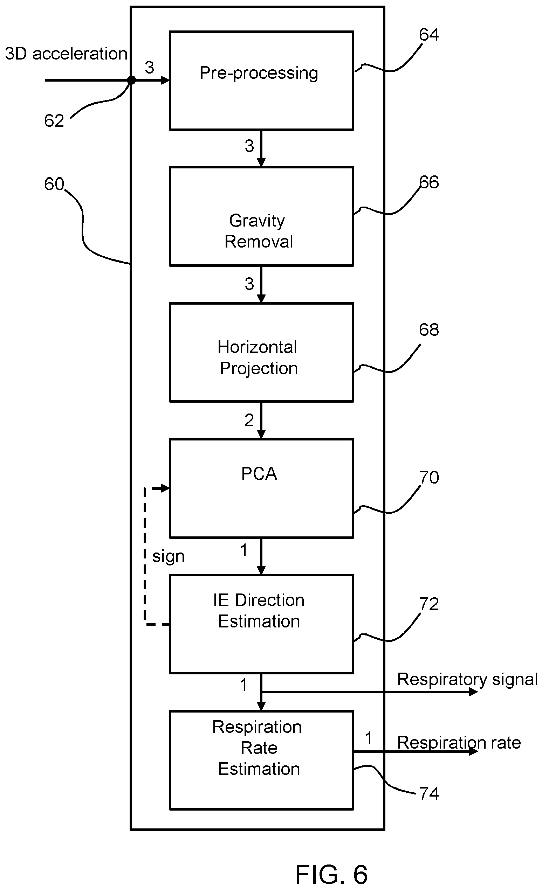

Figure 6 shows the processing steps which are used to process the 3D acceleration data.

The numbers at the input to each processing state show the dimensionality of the signals.

The processing steps are all implemented by a processor 60. The processor 60 receives as input 62 the 3 dimensional (i.e. 3-axis) acceleration signal.

In a first, pre-processing, step 64, the 3D acceleration signal is sampled, for example at 250 Hz. Respiratory patterns for example have signal components up to around 1.0 Hz, so this represents a high degree of over-sampling. Therefore, the signals are also low-pass filtered, for example with a second-order Butterworth filter with a cutoff point of 1 Hz, and are decimated to a lower sampling rate, for example to 16 Hz. The computational complexity of subsequent steps is thereby significantly reduced.

In step 66, the filtered signals are processed to perform isolation of the gravity vector gravity and removal of the gravity vector. The respiratory components in the 3D acceleration signals are relatively small compared to the gravity components. Therefore, it is advantageous first to remove the gravity components.

There are various possible methods for gravity removal.

One possible method is based on a recursive process by which an estimate is recursively adjusted until a match is found. First, gravity is estimated through exponential smoothing:

$$\vec{g_{n+1}} = \alpha \, \vec{a_n} + (1 - \alpha) \, \vec{g_n}$$

In this formula, for time step n, $\vec{g_n}$ is the gravity estimate, and $\vec{a_n}$ is the 3D acceleration. $\alpha$ is a smoothing parameter. It may for example be set such that adaptation to changes in gravity and resilience against artifacts are balanced. Typically the value of $\alpha$ is low, such as below 0.1. A value of 0.0652 in combination with a sampling rate of 16Hz is used in this analysis.

The estimated gravity is subtracted from the 3D acceleration signal, such that a 'zero-gravity' acceleration (with removal of a time-averaged gravity vector) is obtained:

$$\vec{a_n^0} = \vec{a_n} - \vec{g_n}$$

The horizontal projection mentioned above is performed in step 68. As explained above, a respiratory pattern takes place in the horizontal plane (in the transformed coordinate system).

Another possible method may operate on blocks of data, by subtracting the mean acceleration over the block.

The gravity vector is "isolated". It may be removed before the coordinate transformation, but the projection to the horizontal plane after the coordinate transformation also removes the gravity vector. Thus, removal of the gravity vector (in particular a time averaged value) may be implemented in different ways.

The respiratory pattern analysis can thus be restricted to the horizontal plane. The generation of the horizontal projection first involves performing a transformation to the new coordinate system $\{\vec{h_1}, \vec{h_2}, \vec{v}\}$ in which the axes $\vec{h_1}$ and $\vec{h_2}$ lie in the horizontal plane.

The vertical axis $\vec{v}$ can then be discarded.

The first step is to identify the components of the gravity vector $\vec{g}$ in the original accelerometer coordinate system.

This information is already obtained as part of the gravity removal step.

Using $\vec{g} = [g_x, g_y, g_z]^T$, a rotation matrix $\boldsymbol{R}$ is created, such that:

$$\vec{a_h} = \boldsymbol{R} \, \vec{a},$$

wherein $\vec{a}$ is the acceleration in $\{\vec{x}, \vec{y}, \vec{z}\}$ and $\vec{a_h}$ the acceleration in the transformed coordinate system $\{\vec{h_1}, \vec{h_2}, \vec{v}\}$.

The Rotation $\boldsymbol{R}$ corresponds to two successive rotations. First around the x-axis ($\boldsymbol{R_1}$) and then around the y-axis ($\boldsymbol{R_2}$):

$$R = \frac{1}{\rho} R_2 R_1,$$

with:

6

$$R_1 = \begin{bmatrix} \rho & 0 & 0 \\ 0 & g_z & -g_y \\ 0 & g_y & g_z \end{bmatrix} \qquad R_2 = \begin{bmatrix} \rho & 0 & g_x \\ 0 & 1 & 0 \\ -g_x & 0 & \rho \end{bmatrix} \qquad \rho = \sqrt{g_z{}^2 + g_y{}^2}$$

For the subsequent analysis, only the $\vec{h_1}$ and $\vec{h_2}$ components of $\vec{a_h}$ are used, such that the number of dimensions is reduced to two as shown in Figure 6.

Principal Component Analysis is used in step 70. The first objective is to estimate a respiratory signal $\hat{r}$ that resembles the reference signal (r) shown in Figure 5 as much as possible. The signal $\hat{r}$ is one-dimensional, while the acceleration signal is three-dimensional. Hence, the number of dimensions needs to be reduced.

Principal Component Analysis is a well known tool for this type of data processing, and it implements Hebbian Learning. PCA is for example discussed in "Generalizations of Principal Component Analysis, Optimization Problems, and Neural Networks", J. Karhunen and J. Joutsensalo, Neural Networks, Vol. 8, No. 4, pp 549-562, 1995.

In Hebbian learning, neural paths that cause high activations are reinforced through the feedback of the activation. As only one dimension is needed, only the first principal component is required and so-called Oja's rule can be used.

Oja's rule works iteratively. Per time step, one iteration is executed. In each iteration, new a new input sample is projected onto a projection axis $\vec{w}$, such that the one-dimensional signal $\hat{r}$ can be estimated:

$$\hat{r_n} = \vec{w_n} \cdot \vec{a_n}$$

The above formula is a dot-product of two vectors and the result $\hat{r}$ is a scalar (and is therefore one-dimensional). In the feedback loop, the activation $\hat{r}$ is used to update the projection axis $\vec{w}$:

$$\vec{w_{n+1}} = \vec{w_n} + \eta\, \hat{r_n} \left( \vec{a_n} - \hat{r}\, \vec{w_n} \right)$$

The result of the update is that the projection axis $\vec{w}$ turns a small amount into the direction of the new input $\vec{a_n}$. The degree to which this takes place depends on the distance of the point to the axis $\vec{a_n} - \hat{r_n}\vec{w_n}$, the activation $\hat{r}$ and the learning rate $\eta$, which is for example set to 0.001.

This iterative way of updating the projection axis perfectly fits with the iterative way of estimating gravity. Also, Oja's rule is a form of exponential smoothing, in which older values fade out exponentially.

In step 72, based on the one dimensional result $\hat{r}$ of the PCA of step 70, there is estimation of the inspiratory and expiratory directions.

For $\hat{r}$ it is desired that inspirations correspond to positive signal transitions and that expirations correspond to negative signal transitions, just as is the case in the reference signal.

Complying with this choice of directions lowers the estimation error. In addition, changes in the direction (flipping), cause extra transitions, which disturb the respiratory rate estimation.

Principal Component Analysis does not estimate the inspiratory direction; in this aspect it makes an arbitrary choice. For this reason, the estimation of the inspiratory direction is performed, using $\hat{r}$.

A feature that correlates strongly with the proper choice of the inspiratory direction is the skewness of $\hat{r}$. If $\hat{r}$ has the proper direction then the distribution of $\hat{r}$ is right-skewed (the skewness is positive). In this case, the samples in the left part of the distribution (the negative signal values) correspond to the inspiratory pause, in which the diaphragm and intercostal muscles are relaxed. The samples in the right part of the distribution (the positive signal values) correspond to the interval where the lungs are maximally filled.

Thus, a measure of skewness is used to determine the inspiratory direction from the different periods of time of the signal $\hat{r}$.

The signal at the output of step 70 is the same as the respiratory signal that comes out of block 72. Thus, step 72 is transparent to this signal. Step 72 estimates the direction and feeds this back to step 70, where corrections are made by flipping the direction of the projection axis.

Thus, when the direction of the projection axis is set correctly, inspirations correspond to upward transitions of this signal.

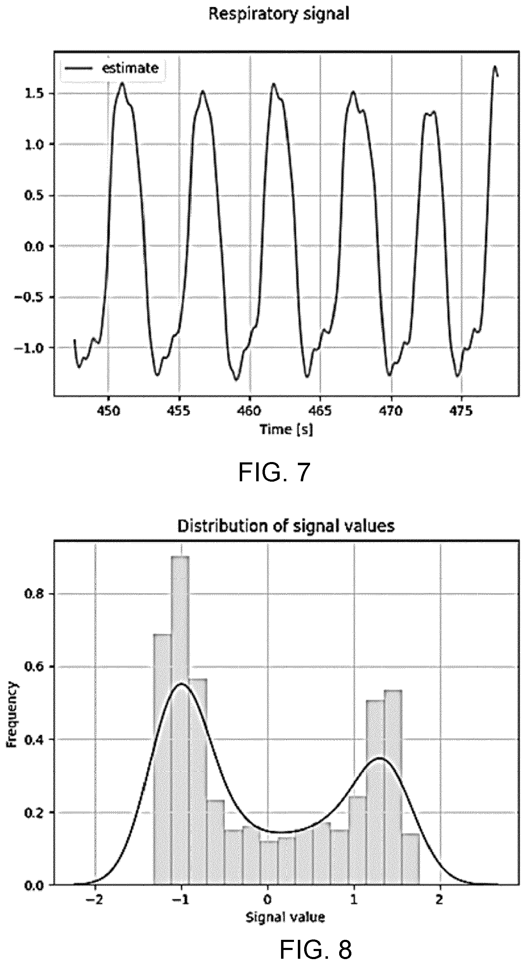

Figure 7 shows an estimate of the respiratory signal. Figure 8 shows the frequency distribution of the estimate. The skewness is 0.3, meaning the distribution is right-skewed.

The definition of skewness is as follows (with s the standard deviation and $\mu$ the mean):

$$g = \frac{\sum_{i=1}^{n}(\hat{r}_i - \mu)^3 / N}{s^3}$$

One approach is a recursive method. Instead of averaging, as in the definition above for skewness, exponential smoothing may be performed. Furthermore, $\hat{r}$ has a zero-mean and is normalized to have its standard deviation equal to one. Because of this, the skewness is approximated by the formula below.

An advantage of this approximation is that it fits very well in the recursive scheme of the other steps.

$$s_{n+1} = \alpha \hat{r}_n^3 + (1 - \alpha) s_n$$

The smoothing parameter $\alpha$ is for example equal to 0.0005. Using this in combination with a sampling rate of 16Hz gives a time constant of roughly 1 minute.

If the skewness $s_n$ is above a threshold (i.e. the distribution is sufficiently right-skewed) then the recursive process can continue without any changes; the projection axis $\vec{w}$ has the right direction and inspirations correspond to positive transitions. However, if the skewness $s_n$ goes below a threshold then the projection axis $\vec{w}$ has the wrong direction.

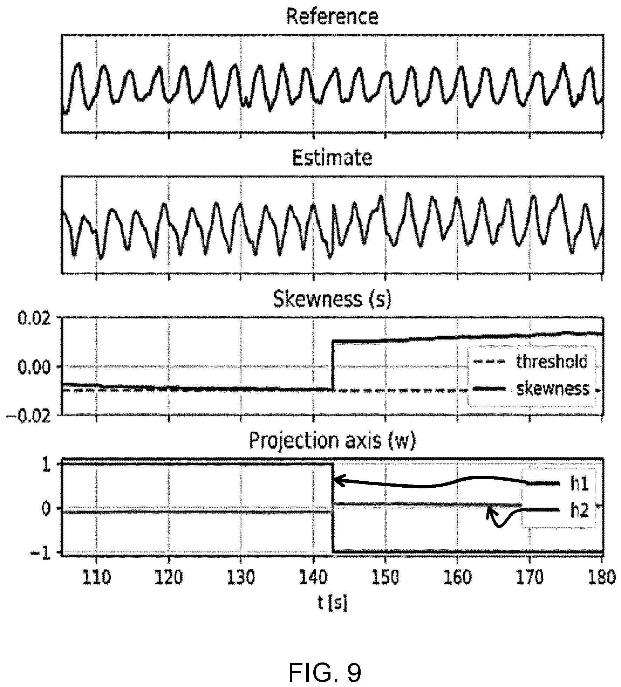

A correction must be made, as shown in Figure 9. Figure 9 shows an example of the correction of the inspiratory and expiratory directions. From top to bottom Figure 9 shows: the respiratory reference (from the RIP band), the respiratory estimate, the skewness with the threshold (dotted), and the projection axis (h1, h2).

Before t=143 s, the sign is inverted and therefore the skewness is decreasing. At t=143, the skewness drops below the threshold, causing the respiratory estimate, the skewness and the projection axis to be inverted.

The correction thus consists of inverting the sign of the projection axis $\vec{w}$, the skewness $s_n$ and the respiratory estimate $\hat{r}_n$. The inversion of the signal of the projection axis $\vec{w}$ is a feedback to the PCA step, which is visible as a dashed arrow in Figure 6.

The inversion of the respiratory estimate $\hat{r}_n$ manifests itself as a discontinuity as shown in the second plot of Figure 9.

Setting the threshold to -0.01 prevents repeated inversion; when the skewness is decreasing, and $s_n$ goes below the threshold, then the sign of $s_n$ is flipped, such that $s_n$ is above the threshold. Due to the flipping, a decreasing trend now becomes an increasing trend, and $s_n$ must change trend and decrease at least by twice the magnitude of the threshold before another inversion occurs Thus, a hysteresis effect is obtained, which prevents successive flipping.

The output of the unit 72 is the respiration signal with 16 Hz sampling rate, i.e. a reconstruction of a breathing signal corresponding to the reference signal shown in Figure 5.

The reference signal (from a RIP band) is a surrogate measurement for respiratory effort. By aiming to reconstruct this reference signal as closely as possible, by minimizing the difference between the respiratory signal from step 72 and the reference, the system generates a respiratory effort signal and a respiration rate.

The respiration rate is estimated in step 74 by detecting peaks in a spectrogram, followed by statistical post-processing.

For this purpose, the respiration signal is down sampled to 4 Hz. This suffices for the purpose of respiration rate analysis. Then, a window is slid over the signal. The window length is for example 30 seconds (120 samples) and the time difference between successive windows may be 5 seconds.

Per window, a Fourier transform is applied (N=256, by zero-padding). The frequency resolution is:

$$\Delta f = \frac{f_s}{2N} 60 = \frac{240}{512} = 0.5 \text{ bpm}$$

For each window, the spectrum is normalized. The normalization constant is chosen such that the peak of a purely sinusoidal signal would have height 1.

The frequency $f_n$ of the highest peak in the spectrum of each window is selected and its height $h_n$ is recorded with it:

$$(f_n, h_n)$$

In the statistical post-processing, the peaks of 12 successive windows are combined into one estimate for the respiration rate, such that for each minute, a respiration rate estimate becomes available. First, peaks with a height above a threshold th are selected. Over these peaks, a weighted mean is taken in which the weights are the peak heights and the values are the respiratory rates of the peaks.

$$f_B = \frac{\sum_{n \text{ for which } h_n > th} h_n f_n}{\sum_{n \text{ for which } h_n > th} h_n}$$

If no peaks exist for which $h_n > th$ then for that minute no respiration rate is determined. The coverage is the number of intervals for which a respiration rate could be determined, divided by the total number of intervals.

The agreement between the estimated respiration rate and the reference has been analyzed and the results are shown in Figure 8.

The three plots in Figure 8 show the difference in respiration rate versus the mean respiration rate, comparing the respiratory rate estimated from the acceleration signal and that estimated from the reference signal. In particular, the horizontal axis in each case shows the mean of the two rates being compared and the vertical axis shows the difference between the two. The dashed lines are at -1.96 SD, 0 and +1.96 SD.

Each dot is a respiration rate over one minute. The dots are taken over all subjects, device positions, and postures and are grouped per posture (supine "sup", left and right). The diagrams are according to Bland-Altman.

The results show close agreement between the respiration rates calculated using the two approaches.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims. (optional)

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to".

Any reference signs in the claims should not be construed as limiting the scope.

**Claims**

1. A processor (60) for determining a breathing rate, comprising:
   an input (62) for receiving a 3-axis acceleration signal, wherein the processor is adapted to:

   (66) isolate a gravity vector from the 3-axis acceleration signal;
   (68) perform a coordinate transformation into a transformed 3-axis coordinate system in which a time-averaged gravity vector is aligned with a first axis of the transformed 3-axis coordinate system;
   (70) perform analysis of the components for the remaining two axes of the transformed 3-axis coordinate system, thereby to derive a 1 dimensional respiratory signal; and
   (74) determine a respiration rate from the 1 dimensional respiratory signal using frequency analysis.

2. The processor of claim 1, adapted to perform pre-processing (64) of the acceleration signal before isolating the gravity vector, wherein the pre-processing comprises sampling and low-pass filtering.

3. The processor of claim 1 or 2, adapted to isolate the gravity vector from the 3-axis acceleration signal by implementing a recursive exponential smoothing function.

4. The processor of any one of claims 1 to 3, adapted to perform the coordinate transformation into the transformed 3-axis coordinate system by deriving a rotation matrix which maps the isolated gravity vector to the first axis.

5. The processor of any one of claims 1 to 3, adapted to perform the analysis to derive the 1 dimensional respiratory signal using principal component analysis or using machine learning.

**6.** The processor of any one of claims 1 to 5, adapted to estimate the inspiratory and expiratory directions from the 1 dimensional respiratory signal.

**7.** The processor of claim 6, adapted to estimate the inspiratory and expiratory directions by (72) determining a skewness value of the 1 dimensional respiratory signal.

**8.** The processor of claim 7, adapted to perform an inversion if needed, such that one type of skewness corresponds to the inspiratory direction and another type of skewness corresponds to the expiratory direction.

**9.** The processor of any one of claims 1 to 8, adapted to determine the respiration rate from the 1 dimensional respiratory signal by applying a Fourier transform over a sliding time window and determining the frequency of the highest peak in the spectrum of each window, wherein the respiration rate is derived from the highest peaks for a set of successive windows

**10.** A system for determining a breathing rate, comprising:

an accelerometer for generating a 3-axis acceleration signal; and
the processor of any one of claims 1 to 9 for processing the 3-axis acceleration signal.

**11.** A method for determining a breathing rate, comprising:

receiving a a 3-axis acceleration signal;
isolating a gravity vector from the 3-axis acceleration signal;
performing a coordinate transformation into a transformed 3-axis coordinate system in which the isolated gravity vector is aligned with a first axis of the transformed 3-axis coordinate system;
performing analysis of the components for the remaining two axes of the transformed 3-axis coordinate system, thereby to derive a 1 dimensional respiratory signal; and
determining a respiration rate from the 1 dimensional respiratory signal using frequency analysis.

**12.** The method of claim 10 or 11, comprising one or more of:

performing pre-processing of the acceleration signal before isolating the gravity vector, wherein the pre-processing comprises sampling and low-pass filtering;
isolating the gravity vector from the 3-axis acceleration signal by implementing a recursive exponential smoothing function;
performing the coordinate transformation into the transformed 3-axis coordinate system by deriving a rotation matrix which maps a time-averaged gravity vector to the first axis; and
performing the analysis to derive the 1 dimensional respiratory signal using principal component analysis or using machine learning.

**13.** The method of any one of claims 11 to 12 comprising estimating the inspiratory and expiratory directions from the 1 dimensional respiratory signal by determining a skewness value of the 1 dimensional respiratory signal.

**14.** The method of any one of claims 11 to 13, comprising determining the respiration rate from the 1 dimensional respiratory signal by applying a Fourier transform over a sliding time window and determining the frequency of the highest peak in the spectrum of each window, and deriving the respiration rate from the highest peaks for a set of successive windows

**15.** A computer program comprising computer program code which is adapted, when said computer program code is run on a computer, to implement the method of any one of claims 11 to 14.

FIG. 1

FIG. 2

FIG. 3

FIG. 4

FIG. 5

FIG. 6

EP 3 973 866 A1

## Respiratory signal

FIG. 7

## Distribution of signal values

FIG. 8

FIG. 9

FIG. 10

Europäisches
Patentamt

European
Patent Office

Office européen
des brevets

# EUROPEAN SEARCH REPORT

Application Number

EP 20 19 8259

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 263 532 A1 (KONINKL PHILIPS ELECTRONICS NV [NL] ET AL.) 22 December 2010 (2010-12-22) | 1-6, 10-12,15 | INV. A61B5/08 A61B5/00 |
| Y | * paragraphs [0027] - [0029], [0031], [0042], [0047], [0050] - [0052], [0064], [0075] - [0078], [0081] * * figure 1 * | 7,8,13 | |
| X | WANG JU ET AL: "In-Vehicle Respiratory Rate Estimation Using Accelerometers", PHEALTH 2019, [Online] 10 June 2019 (2019-06-10), XP055782533, DOI: 10.3233/978-1-61499-975-1-97 Retrieved from the Internet: URL:https://plri.de/content/6-team/thomas-deserno/bibliografieliste/2019_01_Stud_Health_Technol_Inform_In-vehicle_respirato/2019_wang_in_vehicle-respiratory-rate-estimation.pdf> [retrieved on 2021-03-05] * page 99: chapter 2.3 * * figure 2 * | 1,9,11, 14,15 | |
| X | US 2009/062628 A1 (YAMAMOTO MATSUKI [JP] ET AL) 5 March 2009 (2009-03-05) * paragraphs [0061] - [0063], [0105] * * figures 1, 5 * | 1,11,15 | TECHNICAL FIELDS SEARCHED (IPC) A61B |
| Y | Filliben James J. ET AL: "1.3.5.11. Measures of Skewness and Kurtosis" In: "Engineering Statistics Handbook", 27 June 2012 (2012-06-27), XP055782473, Retrieved from the Internet: URL:https://doi.org/10.18434/M32189> * the whole document * | 7,8,13 | |
| A | DE 10 2019 000608 A1 (DRAEGERWERK AG & CO KGAA [DE]) 30 July 2020 (2020-07-30) * paragraph [0067] * | 1-15 | |

The present search report has been drawn up for all claims

| Place of search | Date of completion of the search | Examiner |
|---|---|---|
| The Hague | 5 March 2021 | Worms, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 20 19 8259

This annex lists the patent family members relating to the patent documents cited in the above-mentioned European search report.
The members are as contained in the European Patent Office EDP file on
The European Patent Office is in no way liable for these particulars which are merely given for the purpose of information.

05-03-2021

| Patent document cited in search report | | Publication date | Patent family member(s) | | Publication date |
|---|---|---|---|---|---|
| EP 2263532 | A1 | 22-12-2010 | BR | PI1008187 A2 | 15-09-2020 |
| | | | CN | 102458246 A | 16-05-2012 |
| | | | EP | 2263532 A1 | 22-12-2010 |
| | | | EP | 2437662 A1 | 11-04-2012 |
| | | | JP | 5784590 B2 | 24-09-2015 |
| | | | JP | 2012528657 A | 15-11-2012 |
| | | | RU | 2011152964 A | 20-07-2013 |
| | | | US | 2012065524 A1 | 15-03-2012 |
| | | | WO | 2010140130 A1 | 09-12-2010 |
| US 2009062628 | A1 | 05-03-2009 | US | 2009062628 A1 | 05-03-2009 |
| | | | WO | 2006123691 A1 | 23-11-2006 |
| DE 102019000608 | A1 | 30-07-2020 | DE | 102019000608 A1 | 30-07-2020 |
| | | | WO | 2020156686 A1 | 06-08-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015018752 A

**Non-patent literature cited in the description**

- **J. KARHUNEN ; J. JOUTSENSALO.** Generalizations of Principal Component Analysis, Optimization Problems, and Neural Networks. *Neural Networks,* 1995, vol. 8 (4), 549-562